# EUROPEAN PATENT APPLICATION

(11) **EP 2 985 739 A1**
(43) Date of publication of application: **17.02.2016**
(21) Application number: 15179807.1
(22) Date of filing: 05.08.2015
(51) Int. Cl.: G07C 11/00, G08B 21/00

(54) **SYSTEMS AND METHODS FOR MONITORING HAND HYGIENE PROTOCOL**

(30) Priority: 11.08.2014 US 201414456099
(71) Applicant: Schneider Electric Buildings, LLC, Palatine, IL 60067-7399 (US)
(72) Inventor: ROSEBRAUGH, Warren Dale, Salem, NH 03079 (US); MONROE, Thomas Allen, Raleigh, NC 27614 (US)
(74) Representative: Zerbi, Guido Maria

(57) **Abstract**

Hygiene management systems are provided for managing hygiene compliance. In some embodiments, the hygiene systems can include video cameras to count people entering a managed room. Responsive to each detected entry a timer can be started by the system, which is reset by activation of hygiene stations (e.g., hand washing stations or sanitizer dispensers). If the timers expire without use of the hygiene stations, the system triggers visual and/or audible alarms to remind people to use the hygiene stations.

## Description

### BACKGROUND

### Technical Field

The technical field of this disclosure relates generally to health management monitoring systems and, more particularly, to systems and methods that monitor hand hygiene protocols in building management systems.

### Background Discussion

Managing patient health in a hospital setting is undergoing increasing focus. Various initiatives in the healthcare setting are targeting reimbursements by healthcare payment providers (e.g., provided by Medicaid and other third party payment providers). For example, medical expenses to cure hospital induced infections may no longer be reimbursed. Various conventional systems exist that attempt to track individuals' use of hygiene systems (e.g., hand sanitizer dispensers, soap stations, etc.).

### SUMMARY

It is realized that some of the conventional approaches for tracking individuals and ensuring hygiene compliance fail to include large groups of people who bring and/or transfer infection vectors into the hospital setting. For example, conventional systems based on radio frequency identifiers ("RFID") can only track and ensure compliance for those individuals who have been issued RFID devices. Other systems track only the use of hand washing stations, but provide no information on individuals entering and/or leaving a room. Accordingly, various aspects and embodiments are directed to systems and methods for effectively managing healthcare hygiene compliance. Some embodiments incorporate video monitoring of individual rooms to detect any entry and/or exit of any individual (e.g., patients, healthcare worker, visitors, etc.).

According to one embodiment, each count of an entry or exit triggers timer operations during which the system monitors hygiene stations (e.g., soap dispenser, hand sanitizer dispense, etc.) either inside or proximate to the managed room. If no activity occurs at the hygiene stations, the timer operations expire causing the system to trigger one or more alarms. In some examples, the alarm can include a visual display (e.g., turning lights on/off in the room or proximate to the room, flashing lights, among other options). In other examples, multiple alarm levels can be triggered, the first including visual displays. A second alarm level can be triggered if another timer or time period expires. The second level alarm can include, for example, audio messages requesting compliance with hygiene protocols.

According to one aspect, a system for managing hygiene compliance is provided. The system comprises at least one processor operatively connected to a memory, the at least one processor configured to instantiate a plurality of system components, a detection component configured to detect at least one person within a managed room, a timer component configured to manage a threshold time period responsive to detecting the at least one person within the managed room, a hygiene monitoring component configured to receive activation information from one or more hygiene stations, and an alert component configured to trigger an alarm based on activation information associated with the one or more hygiene stations and the threshold time period.

According to one embodiment, the timer component is further configured to manage multiple time periods associated with each entry into the managed room. According to one embodiment, the timer component is further configured to communicate a first signal to the alert component upon expiration of a first time period. According to one embodiment, the alert component is further configured to trigger a first alarm responsive to expiration of the first time period. According to one embodiment, the timer component is further configured to communicate a second signal to the alert component upon expiration of a second time period.

According to one embodiment, the alert component is further configured to trigger a second alarm responsive to expiration of the second time period. According to one embodiment, the alert component is further configured to trigger one of or both of a visual and audible alarm responsive to expiration of the threshold time period without activation of the one or more hygiene stations. According to one embodiment, the audible alarm includes a reminder message announced in multiple languages. According to one embodiment, the timer component is further configured to trigger respective timers associated with each person entering the managed room. According to one embodiment, the alert component is further configured to trigger an alarm responsive to failing to detect an activation of the one or more hygiene stations within a threshold time period associated with each respective timer.

According to one aspect a method for managing hygiene compliance is provided. The method comprises detecting, by a detector, at least one person within a managed room responsive to access information, managing, by a timer, a threshold time period responsive to detecting the at least one person within the managed room, monitoring one or more hygiene stations for activation information, and triggering an alarm based on the activation information associated with the one or more hygiene stations and the threshold time period.

According to one embodiment, managing the threshold timer period includes managing multiple time periods associated with each entry into the managed room. According to one embodiment, the method further comprises generating a first signal upon expiration of a first time period. According to one embodiment, the method further comprises triggering a first alarm responsive to generating the first signal. According to one embodiment, the method further comprises generating a second signal upon expiration of a second time period. According to one embodiment, the method further comprises triggering a second alarm responsive to generating the second signal.

According to one embodiment, the method further comprises triggering one of or both of a visual and audible alarm responsive to exceeding the threshold time period without activation of the one or more hygiene stations. According to one embodiment, triggering the alarm includes announcing a reminder message in multiple languages. According to one embodiment, the method further comprises triggering respective timers associated with each person entering the managed room. According to one embodiment, triggering the alarm includes triggering an alarm responsive to failing to detect an activation of the one or more hygiene stations within a threshold time period associated with each respective timer.

According to one aspect, at least one programmable logic circuit for managing hygiene compliance is provided. The at least one programmable logic circuit is configured to detect at least one person entering a managed room responsive to access information, manage a threshold time period responsive to the access information, monitor activation information from one or more hygiene stations, and trigger an alarm based on activation information associated with the one or more hygiene stations and the threshold time period.

According to one aspect, a system for managing hygiene compliance is provided configured to detect at least one person entering a managed room responsive to access information, manage a threshold time period responsive to the access information, monitor activation information from one or more hygiene stations, and trigger an alarm based on activation information associated with the one or more hygiene stations and the threshold time period.

According to one aspect, a non-transitory computer readable medium is provided. The non-transitory computer readable medium having stored thereon sequences of instruction for managing hygiene compliance including instructions that cause at least one processor of a computer system to detect people entering a managed room responsive to access information, manage a threshold time period responsive to the access information within the managed room, monitor activation information from one or more hygiene stations, and trigger an alarm based on activation information associated with the one or more hygiene stations and the threshold time period.

Other aspects, embodiments and advantages of these exemplary aspects and embodiments, are discussed in detail below. Moreover, it is to be understood that both the foregoing information and the following detailed description are merely illustrative examples of various aspects and embodiments, and are intended to provide an overview or framework for understanding the nature and character of the claimed aspects and embodiments. Any embodiment disclosed herein may be combined with any other embodiment. References to "an embodiment," "an example," "some embodiments," "some examples," "an alternate embodiment," "various embodiments," "one embodiment," "at least one embodiment," "this and other embodiments" or the like are not necessarily mutually exclusive and are intended to indicate that a particular feature, structure, or characteristic described in connection with the embodiment may be included in at least one embodiment. The appearances of such terms herein are not necessarily all referring to the same embodiment or example.

### BRIEF DESCRIPTION OF DRAWINGS

Various aspects of at least one embodiment are discussed below with reference to the accompanying figures, which are not intended to be drawn to scale. The figures are included to provide an illustration and a further understanding of the various aspects and embodiments, and are incorporated in and constitute a part of this specification, but are not intended as a definition of the limits of any particular embodiment. The drawings, together with the remainder of the specification, serve to explain principles and operations of the described and claimed aspects and embodiments. In the figures, each identical or nearly identical component that is illustrated in various figures is represented by a like numeral. For purposes of clarity, not every component may be labeled in every figure. In the figures:
FIG. 1 is a block diagram of an example hygiene management system, according to one embodiment;
FIG. 2A-C illustrate example elements of hygiene management systems, according to some embodiments;
FIG. 3 is a flow diagram illustrating a process for managing hygiene protocols, according to one embodiment;
FIG. 4 is a screen capture of a user interface, according to one embodiment; and
FIG. 5 a schematic diagram of an exemplary computer system that may be specially configured to perform processes and functions disclosed herein.

### DETAILED DESCRIPTION

According to one aspect, conventional systems for tracking hygiene compliance are augmented to manage hygiene tracking and/or compliance based on detecting any and all individual visits to a room. According to one embodiment, monitoring systems (e.g., video, motion, etc.) can be used to count entry and exit to rooms in a managed building (e.g., patient rooms in a medical care setting). The monitoring triggers a hygiene event including a timer to track time between entry/exit and activation of a hygiene station (e.g., hand wash station, soap dispenser, hand sanitizer dispenser, etc). According to various embodiments, integrating entry monitoring into hygiene management provides an improvement over existing systems by creating a more efficient, inclusive, and accurate hygiene management system. In one example, video monitoring eliminates the need for tracking hardware, which in conventional approaches has to be issued, for example, to medical staff.

According to other embodiments, additional efficiencies are realized in the system based on coupling individual counts with hygiene station activation. Namely, accurate (e.g., counting of all individuals entering and existing and not just staff) and efficient management of hygiene protocols can be realized using existing building management systems augmented as discussed herein. In other embodiments, enforcement of hygiene protocols can be extended using the management system outside conventional boundaries, for example, defined by the need to manage or distribute hardware to the people being managed.

Examples of the methods and systems discussed herein are not limited in application to the details of construction and the arrangement of components set forth in the following description or illustrated in the accompanying drawings. The methods and systems are capable of implementation in other embodiments and of being practiced or of being carried out in various ways. Examples of specific implementations are provided herein for illustrative purposes only and are not intended to be limiting. In particular, acts, components, elements and features discussed in connection with any one or more examples are not intended to be excluded from a similar role in any other examples.

Also, the phraseology and terminology used herein is for the purpose of description and should not be regarded as limiting. Any references to examples, embodiments, components, elements or acts of the systems and methods herein referred to in the singular may also embrace embodiments including a plurality, and any references in plural to any embodiment, component, element or act herein may also embrace embodiments including only a singularity. References in the singular or plural form are not intended to limit the presently disclosed systems or methods, their components, acts, or elements. The use herein of "including," "comprising," "having," "containing," "involving," and variations thereof is meant to encompass the items listed thereafter and equivalents thereof as well as additional items. References to "or" may be construed as inclusive so that any terms described using "or" may indicate any of a single, more than one, and all of the described terms.

### Hygiene Management System

Some embodiments implement a hygiene management control system that provides for and can automatically enforce hygiene protocols for managed rooms according to specified timing criteria. In one embodiment, video monitoring identifies entry of a person into the patient's room and each entry is used to enforce hygiene protocols. For example, in the healthcare setting, medical personnel can be trained to wash their hands upon entry to a patient's room. Further, all staff can be advised of hygiene protocols that require hand washing/sanitizing between each patient. According to some embodiments, the system can be configured to monitor for hygiene activity, for example, at hand washing stations or hand sanitizer dispensers. If no activity is detected by the system, the system can trigger visual alarms to remind the person to use the washing station or hand sanitizer. If no activity is still detected after another time period, additional alarms, including verbal warnings, can be executed. In some examples, verbal warnings can be announced in multiple languages to ensure compliance. In further embodiments, the hygiene management system couples information on entry/exits to a room with activations of hygiene stations enabling accurate and efficient tracking of compliance information.

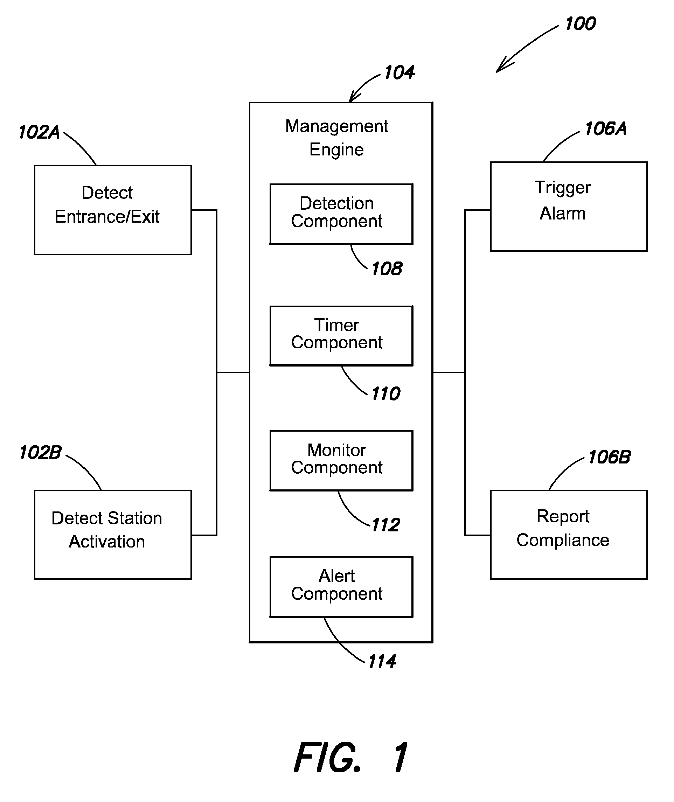

FIG. 1 illustrates one embodiment of a hygiene management control system 100. The hygiene management system 100 can be configured to integrate with existing building management systems ("BMS") to manage hygiene protocols. For example, BMS systems exist that facilitate management of an environment within a building and/or rooms within the building. In some examples, BMS system can control light fixtures, speakers, and/or other systems within a room. Most BMS systems also provide for timing control, for example, associated with heating, cooling, and/or lighting systems. In some embodiments, the hygiene management system can use and/or trigger timing circuits or components of the BMS systems responsive to entry/exit information. In other embodiments, the hygiene management system can include timer circuits/components dedicated to executed hygiene protocols.

According to one embodiment, if no activity occurs at a hygiene station, the timer can trigger any type of alarm to remind people, for example, to wash or sanitize. In some embodiments, the hygiene management control system 100 can be integrated into or with BMS systems so that the hygiene management system can control any system or use available component within the BMS system.

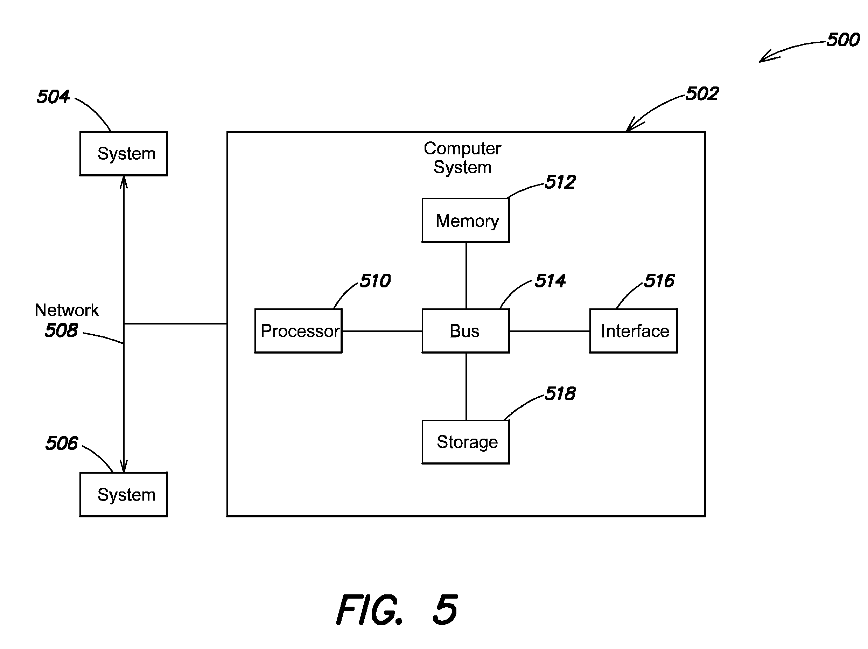

Elements of the system 100 can be provided using a specially configured computing system such as the computer system 500 and/or 502 described with reference to FIG. 5. In one example, the system 100 can include a management engine 104 configured to execute the functions, processes, and/or operations discussed herein. In one example, the management engine 104 can be executed on the computer system (e.g., 500 and/or 502) to provide the functions and operations discussed.

In other embodiments, the management system 100 and/or engine 104 can include additional components executed on the computer system to perform specific operations. For example, the system and/or engine can instantiate specific components configured to execute various operations on the system. In some implementations, the system 100 and/or engine 104 can be configured to communicate with existing BMS systems, to trigger alarms (e.g., visual and/or auditory alarms - at 106A) regarding hygiene protocol enforcement.

As shown in FIG. 1, the management system 100 and/or management engine 104 receives input (e.g., 102A - detect entrance/exit) from surveillance systems (e.g., video monitors, motion detectors, electronic eyes, etc.). According to one embodiment, the system 100 includes a camera mounted above a door to a room in managed building. In some embodiments, the building being managed is in a healthcare environment. The video camera can be configured to detect people as they enter and exit the room through video analytics. In other embodiments, people are detected based on the specific detector being used (e.g., video analytics, motion analytic, interruption of lights beams, etc.). In one example, video information can be captured and communicated (e.g., 102A - detect entrance/exit) to the system 100. In some embodiments, the system 100 and/or engine 104 can analyze the video information to identify people entering and/or exiting the patient's room. In some examples, each entry and/or exit can be used to increment or decrement a count of visitors. In further examples, the count can be based on all people who enter and exit the room, like clinical staff, other hospital staff, patient, family, and visitors.

In some embodiments, video information can be detected and communicated to the system 100 at 102A. In further embodiments, the system 100 and/or engine 104 can include specialized components for processing and identifying individuals entering and/or exiting the managed room (e.g., patient room). In one embodiment, the system and/or engine includes a detection component 108 configured to receive video information and analyze the video data to identify persons entering and exiting a room. In yet other embodiments, the system 100 can be connected to intelligent detector sub-systems (not shown - e.g., video sub-systems), which are configured to capture information (e.g., video, motion, infra-red, etc.) and process the information to identify people entering and exiting the managed room. The intelligent sub-systems can be configured to communicate entrance/exit count information after processing. In some examples, the information communicated at 102A can specify a detected entrance or egress of a person, and the detection component 108 can be configured to increment or decrement a counter accordingly.

Responsive to detecting an entrance and/or incrementing, for example, a person counter, the system 100 and/or engine 104 can be configured to trigger a timer. In some embodiments, the system 100 and/or engine 104 includes a timer component 110 configured to trigger a timer responsive, for example, to a person entering a managed room. The timer can be set to any length of time based on system parameters. In some embodiments, the specific length of time being tracked can be variable. Regardless of the time period set, if the time period expires without any activity at a hygiene station the system 100 and/or engine 104 is configured to trigger an alarm (e.g., at 106A) to notify the individual of the need to use the hygiene station(s).

In some embodiments, the timer component 110 is configured to count down from a specified time and notify an alert component 114 upon expiration of the specified time. In other embodiments, the timer component 114 can be configured to track elapsed time and determine whether a set time limit has been exceeded. The timer component can include multiple timers. For example, each entry into a managed room can trigger a separate timer. In another example, a first timer can be associated with a first alert (e.g., flashing lights), while further failure to activate a hygiene station can trigger a second alert (e.g., audio warning) based on another elapsed time period beyond the first time period.

According to some embodiments, the system 100 and/or engine 104 can also include a monitoring component 112 configured to monitor activity at hygiene stations within or nearby a managed room. For example, sinks and/or soap dispensers can be equipped with sensors configured to determine when the soap dispenser is activated or the sink used. In another example, a hand sanitizer dispenser can include sensors to report on activation of the dispenser. The monitoring component 112 can be configured to capture activation and/or use information from the sensors. Responsive to receiving activation information, the monitoring component 112 can be configured to reset a timer or timer process executed by the timer component 110. For example, when a person entering the managed room washes their hands in the sink - the detection component identifies the entry, the timing component starts a timer, responsive to using the sink, the monitoring component terminates the timer or prevents further action by the system 100 (e.g., no alarms are triggered). If the person does not use the sink or use a hand sanitizer dispenser, then the system 100 triggers alarms.

In some embodiments, the system 100 and/or engine 104 can include an alert component 114 configured to trigger alarms in the managed room. In some examples, the alert component can be configured to communicate with a BMS system to trigger lighting changes in the room, or flash a light near, on, or within a hygiene station. In other examples, the alert component 114 can be configured to manipulate environmental controls in the room (e.g., lights) so that the alert component 114 can directly trigger an alarm within the managed room.

According to one embodiment, the alert component 114 can be configured with multiple alarm levels. In one example, the timer component 110 is configured to trigger a first level alarm (e.g., by communicating to the alert component 114) based on the elapse of a first time period. The timer component 110 can also be configured to track elapsed time for a second level alarm. If a second time period is exceeded without a hygiene station activation, the timer component can trigger a second level alarm (e.g., through the alert component 114). According to various embodiments, the alert component 114 is configured with multiple levels of alarms. A first level alarm can include activation/deactivation of lighting in the managed room and/or dimming or brightening of lights. The first level alarm can also include triggering of flashing lights at the hygiene station(s). In some embodiments, a second level alarm can include audio messages. For example, the system can trigger playback of an audio recording providing a reminder that hand washing/sanitizing is necessary to preserve health. In further examples, the system 100 can be configured to communicate second level alarm messages in multiple languages to ensure understanding and compliance. In other embodiments, multiple alarms can be executed by the system (e.g., visual and audio alarms) in response to one timer or more than one timer.

In further embodiments, the system 100 and/or engine 104 can be configured to track hygiene protocol compliance. In various embodiments, the system 100 is configured to count each entry event (e.g., each person entering and/or exiting a room) and count each activation of a hygiene station. The system 100 can include a database (or other storage system) configured to store the count associated within entries and activations. In some examples, counts of activations can be specific to the stations being used. Sanitizers can be counted separately from soap dispenser activations, which can be counted separately from other hygiene stations (e.g., UV light station, etc.).

In some embodiments, the system can include interfaces for accessing compliance information. In one example, the system can calculate compliance ratios based on the number of persons entering a room and the number of activations of any hygiene station. In another example, the system can track and store information on alarm activation (e.g., in the database). The alarm activation can be used in other embodiments to provide information on compliance. In further examples, alarm information can be combined with entry and/or activation information to provide information on compliance. In various embodiments, system administrators and/or users can access the system and/or database to view information on hygiene compliance. In some examples, the database can be accessed over a communication network (e.g., the Internet). User interfaces configured to display compliance information can also be rendered on client computer systems.

### Example Environment

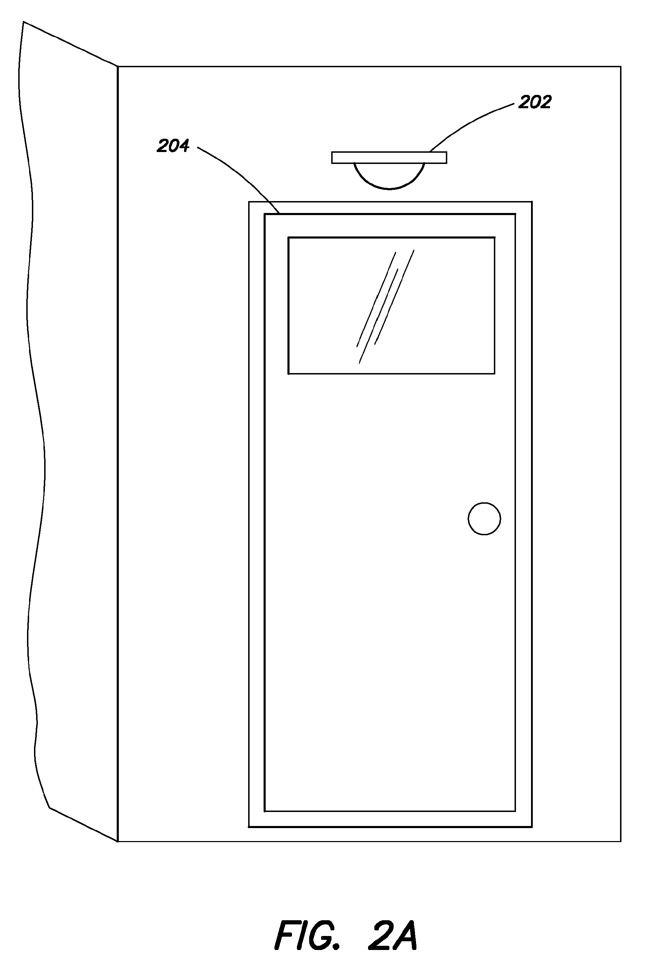

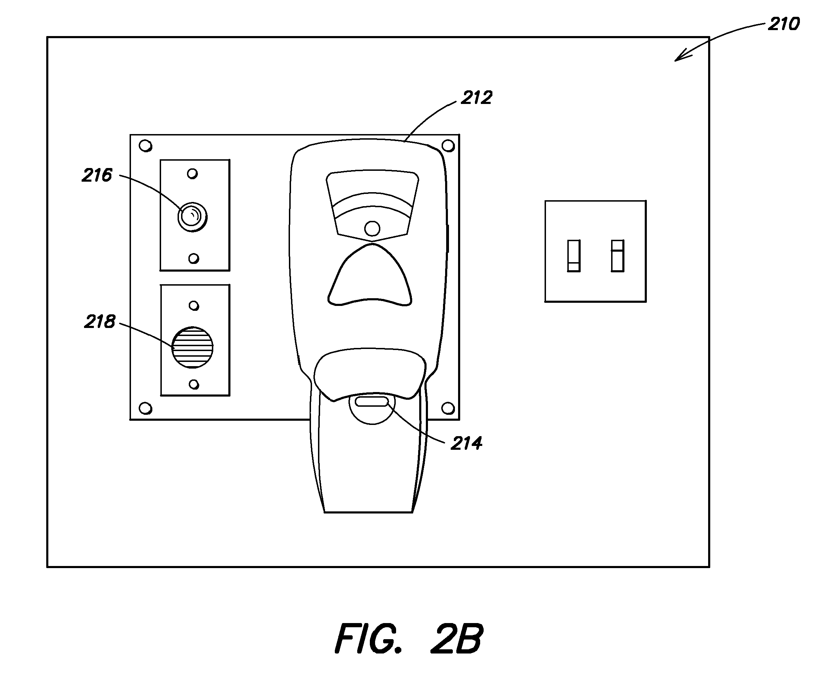

According to some embodiments, the management system 100 can be used to manage patient rooms in a health care setting. The managed room can include patient rooms, visitor rooms, or any room where patients can be exposed to infection, among other examples. According to one embodiment, the system (e.g., 100) can include cameras mounted above patients' rooms. Fig. 2A illustrates an example camera 202 mounted above a door 204 to a patient room. Fig. 2B illustrates an example hygiene station 210. The example hygiene station 210 includes a hand sanitizer dispenser 212 with an activation sensor 214. Responsive to activation, the hand sanitizer dispenser can communicate activation information to the system (e.g., 100) or various components (e.g., monitor component 112). As discussed, as each person enters or exits a managed room an individual timer can be started. In one example, the system can use one or more programmable logic controllers (PLC) already present in the managed room. BMS system use PLCs to control respective BMS functions. In some embodiments, the PLC timers came be set to any predetermined period of time and during this period, the system monitors for any input to the PLC, for example, from an infrared switch (e.g., 214) monitoring hand sanitizer dispenser.

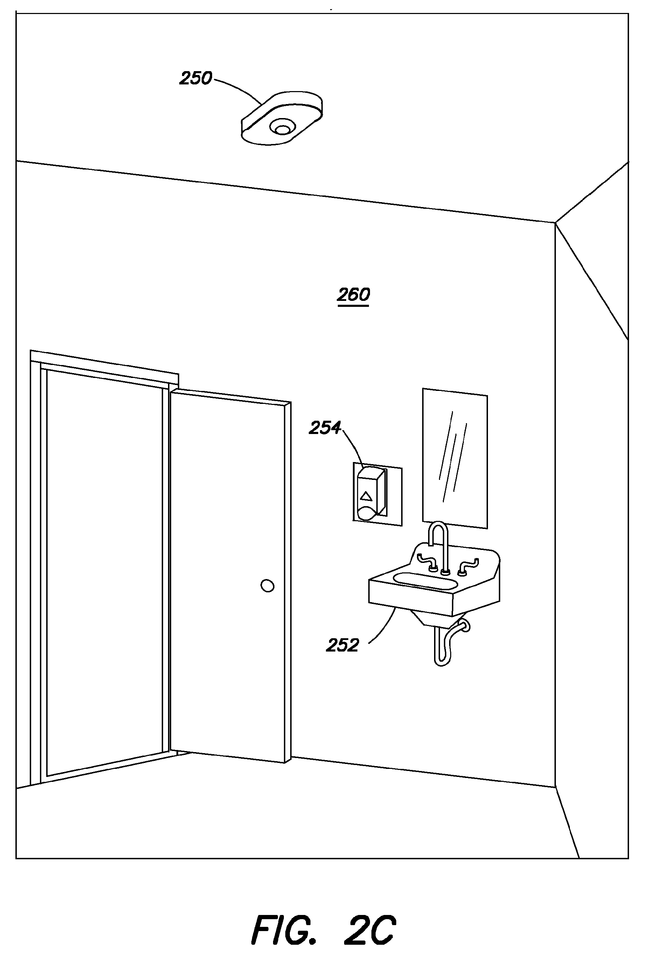

If the period of time expires, then a visual warning (e.g., flashing of light 216) can be displayed based on an output of the PLC. If after another predetermined period of time the dispensers (e.g., 212) are still not depressed/activated a verbal warning can be sounded in one or more languages (e.g., over speaker 218). According to other embodiments, the process or sequencing of detect entry, start timer, trigger one or more alarms (e.g., upon expiration of the timer), can be executed in different order. In further embodiments, the process and/or sequencing can be tailored specific to each patient room. In some embodiments, the system can also detect people existing from a managed room and trigger hygiene alert/alarms as individuals exit a managed room and fail to use hygiene stations. Fig. 2C illustrates another example installation of a hygiene management system (e.g., 100). The system includes a video camera 250 configured to detect persons entering and/or exiting a managed room, for example, a patient room 260. Once an entry event is detected (e.g., persons entering or exiting the room), the system monitors activation of any hygiene station. For example, a hygiene station can include a sink 252 and/or soap dispenser 254. Either or both of the sink and soap dispenser can include activation sensors configured to provide activation information to the system. In addition to deactivating timers based on sensor information, the system can track compliance and entry events. For example, data on compliancy can be compiled for each room, site, each building, zone, department, or at the enterprise level (among other options) and reports can be generated and/or accessed from the system. In some examples, existing BMS system elements can be used to provide access to compliance information, including, for example, network connectivity. In some examples, data on hygiene protocol compliance can be displayed on live dashboards throughout a building and/or facility.

According to some aspects, hygiene management control systems based on video monitoring of entry events achieve a variety of benefits over conventional approaches. According to some examples, video based control systems eliminate the need for specialized tracking hardware (e.g., RFID device). Additionally, video capture of entry events is personnel agnostic. The system identifies and accounts for all people entering, for example, a patient room and not just medical staff. Various embodiments are configured to detect entry by the patient, family, and all visitors. Eliminating the need for additional hardware achieves additional benefits in reducing complexity and eliminating any additional cost per person for managing hygiene. Further embodiments achieve hygiene management without administration of users. In some examples, the system eliminates drawbacks of conventional approach by eliminating badges/cards to purchase and distribute, activate, and/or manage (e.g., including replacing lost cards). In yet other examples, the system can leverage existing infrastructure. Visual and audio alarms can be triggered by the system using existing BMS systems. Further, the visual and audio alarms are specially configured to promote hygiene policy rather than just tracking compliance. Other options include providing audio alarms with policy numbers, best practice commentary, multiple languages, among other potential options.

According to one embodiment, the system can include a privacy mode where image capture by the video cameras can be de-activated in order to maintain patient privacy while still counting entry events. In one example, captured video data is analyzed as the data is captured, and the camera/system only uses analyzed video information indicating a person has entered and/or exited a room. In other examples, video images are not stored by the system when in privacy mode.

In further embodiments, the system can be programmed individually by building and/or room. In some examples, the system can analyze hygiene compliance in rooms are not managed (i.e. that do not trigger alarms) to provide baseline levels against which to measure/evaluate the true impact alerts make on behavior. According to some embodiments, the system uses programmable logic controllers to execute the sequences of operations at the room level, enabling "in the field" future enhancements.

Various process flows and/or sequences of operations can be executed by hygiene management systems. In one example, when a person enters the room the camera detects the entry event triggering an entry contact configured to start, for example, a five second timer. In conjunction with starting the timer, the system can increment an entry counter based on the entry event. If a hygiene station (e.g., soap dispenser or sanitizer dispenser) does not detect that the person has activated the hygiene station within the five second timer: the system can trigger a visual indicator (e.g., light on the hand sanitizer dispenser) and increment a visual indicator counter. If after ten seconds, for example, the hygiene stations has still not been activated, the system can trigger an audible indicator (e.g., speaker) to communicate an audible message. In one example, the audible message is a pre-recorded message "please wash your hands," which can be played in multiple languages.

Responsive to triggering the audible indicator, the system can increment an audible counter. If after twenty seconds, for example, the hygiene station has not been activated, the system can increment a non-compliance counter and turn off any activate indicator (e.g., visual indicator or audible indicator). If the hygiene station (e.g., soap dispenser and/or sanitizer dispenser) is used before the 20 second timer, the system stops any timer, and increments an activation counter for the hygiene station. In some examples, the system can include a counter for any one, two, three, or more of available hygiene devices (e.g., a soap dispenser and a sanitizer dispenser), and increment the respective counter accordingly. If any indicator is active (e.g., visual or audible), the system will also deactivate the indicator responsive to hygiene station activation. In some embodiments, the system is configured to deactivate any timer if the person enters and exits the room (e.g., before the five second timer expires). In some embodiments, data collection can occur at end of day, at which time the system is configured to capture the information in each counter into a log (e.g., a compliance log). Once the data is captured all counters are reset to zero.

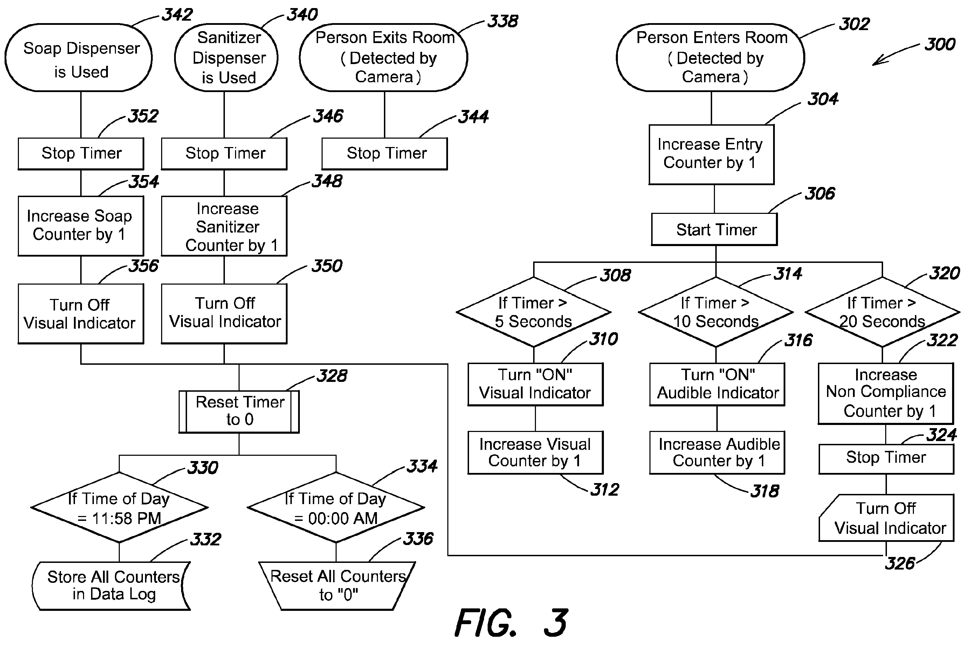

Fig. 3 illustrates an example process flow 300 for managing hygiene protocols. The process 300 begins at 302 with a person entering a managed room. The entry event at 302 can be detected by a camera. At 304, an entry counter is incremented and at a timer is triggered at 306. Once the timer exceeds a first threshold (e.g., five seconds at 308) a visual indicator is turned on at 310 and a visual counter is incremented at 312. Once the timer exceeds ten seconds 314, an audible indicator is turned on at 316 and an audible counter is incremented at 318. Once the timer exceeds twenty seconds at 320, a non-compliance counter is incremented at 322, the timer is stopped at 324 and the visual indicator and/or audible indicator is turned off at 326. At 328, the timer can be reset to zero. According to some embodiments, each entry can trigger process 300, and individual timers associated with each entry can be maintained during respective executions of process 300. In some embodiments, execution of 300 can conclude with end of day accumulation of data. For example, if a current time indicates end of day at 330 all counter data is stored in a data log at 332. A short time later at 334 all counters are reset to zero values at 336.

According to some embodiments, process 300 can be interrupted by interrupt events (e.g., 338, 340, and 342). For example, if the person exits the room at 338, the timer started at 306 is stopped at 344. If at 340, the hand sanitizer dispenser is used, the timer is stopped at 346, and a sanitizer use counter is incremented at 348, if the visual indicator and/or audible indicator are active they are turned off at 350. Likewise, if at 342 the soap dispenser is used, the timer is stopped at 352, the soap use counter incremented at 354 and if the visual and/or audible indicators are active they are turned off at 356. Timers are then reset at 328 and end of day processing continues (e.g., at 330-336).

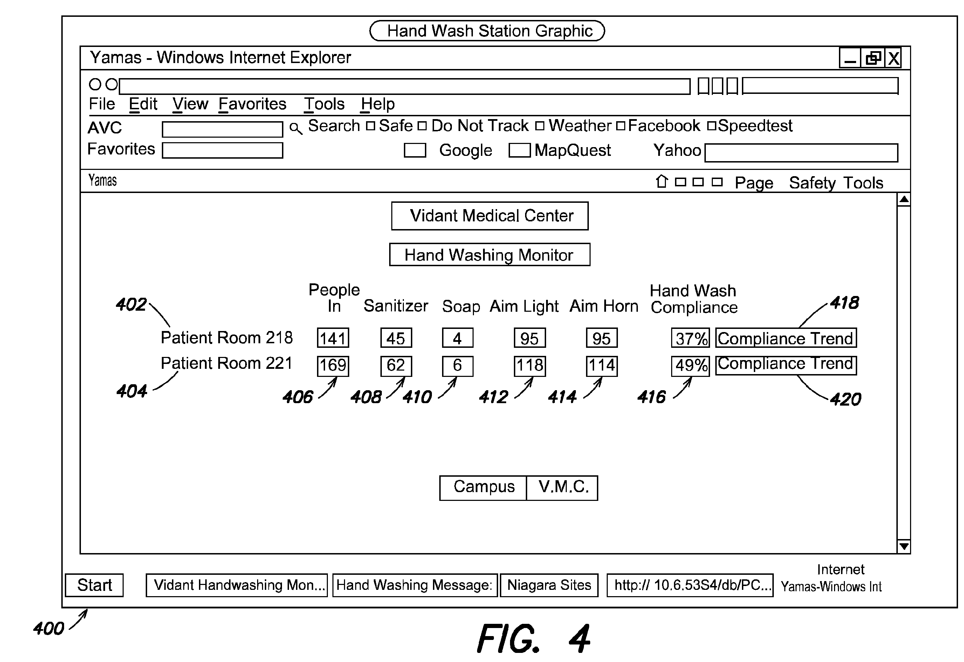

Fig. 4 is an example user interface 400 configured to display hygiene compliance information accumulated by the system (e.g., 100). Data can be stored and accessed in the user interface based on room, building, enterprise, etc. Shown in interface 400 is information on patient rooms "218" (at 402) and "221" (at 404). The display provides access to the data accumulated on entry events (e.g., "people in 141" at column 406; sanitizer counter at column 408; soap counter at 410; visual alarm counter at 412; audible alarm counter at 414; and the percent compliance at column 416. Historical data can be accumulated and plotted to display graphical trends in compliance (e.g., accessed by selecting 418 or 420 for respective rooms).

### Example Computer System

As discussed above with regard to FIG. 1, various aspects and functions described herein may be implemented as specialized hardware or software components executing in one or more computer systems. There are many examples of computer systems that are currently in use. These examples include, among others, network appliances, personal computers, workstations, mainframes, networked clients, servers, media servers, application servers, database servers and web servers. Other examples of computer systems may include mobile computing devices, such as cellular phones and personal digital assistants, and network equipment, such as load balancers, routers and switches. Further, aspects may be located on a single computer system or may be distributed among a plurality of computer systems connected to one or more communications networks.

For example, various aspects and functions including identifying entry into a managed room, triggering timers, activating alarms, and monitoring hygiene stations, may be distributed among one or more computer systems configured to provide a service to one or more client computers, or to perform an overall task as part of a distributed system. Additionally, aspects may be performed on a client-server or multi-tier system that includes components distributed among one or more server systems that perform various functions. Consequently, examples are not limited to executing on any particular system or group of systems. Further, aspects and functions may be implemented in software, hardware or firmware, or any combination thereof. Thus, aspects and functions may be implemented within methods, acts, systems, system elements and components using a variety of hardware and software configurations, and examples are not limited to any particular distributed architecture, network, or communication protocol.

Referring to FIG. 5, there is illustrated a block diagram of a distributed computer system 500, in which various aspects and functions are practiced. As shown, the distributed computer system 500 includes one more computer systems that exchange information. More specifically, the distributed computer system 500 includes computer systems 502, 504 and 506. As shown, the computer systems 502, 504 and 506 are interconnected by, and may exchange data through, a communication network 508. For example, a hygiene management system and/or a management engine can be implemented on 502, which can communicate with a BMS system (e.g., implemented on 506), which operate together to provide hygiene management functions as discussed herein. In other embodiments, hygiene management system and/or engine can include the BMS system, and the functions performed can be implemented by 502 or distributed between 502-506.

In some embodiments, the network 508 may include any communication network through which computer systems may exchange data. To exchange data using the network 508, the computer systems 502, 504 and 506 and the network 508 may use various methods, protocols and standards, including, among others, Fibre Channel, Token Ring, Ethernet, Wireless Ethernet, Bluetooth, IP, IPV6, TCP/IP, UDP, DTN, HTTP, FTP, SNMP, SMS, MMS, SS7, JSON, SOAP, CORBA, REST and Web Services. To ensure data transfer is secure, the computer systems 502, 504 and 506 may transmit data via the network 508 using a variety of security measures including, for example, TLS, SSL or VPN. While the distributed computer system 500 illustrates three networked computer systems, the distributed computer system 500 is not so limited and may include any number of computer systems and computing devices, networked using any medium and communication protocol.

As illustrated in FIG. 6, the computer system 502 includes a processor 510, a memory 512, a bus 514, an interface 516 and data storage 518. To implement at least some of the aspects, functions and processes disclosed herein, the processor 510 performs a series of instructions that result in manipulated data. The processor 510 may be any type of processor, multiprocessor or controller. Some exemplary processors include commercially available processors such as an Intel Xeon, Itanium, Core, Celeron, or Pentium processor, an AMD Opteron processor, a Sun UltraSPARC or IBM Power6+ processor and an IBM mainframe chip. The processor 510 is connected to other system components, including one or more memory devices 512, by the bus 514.

The memory 512 stores programs and data during operation of the computer system 502. Thus, the memory 512 may be a relatively high performance, volatile, random access memory such as a dynamic random access memory (DRAM) or static memory (SRAM). However, the memory 512 may include any device for storing data, such as a disk drive or other non-volatile storage device. Various examples may organize the memory 512 into particularized and, in some cases, unique structures to perform the functions disclosed herein. These data structures may be sized and organized to store values for particular data and types of data.

Components of the computer system 502 are coupled by an interconnection element such as the bus 514. The bus 514 may include one or more physical busses, for example, busses between components that are integrated within the same machine, but may include any communication coupling between system elements including specialized or standard computing bus technologies such as IDE, SCSI, PCI and InfiniBand. The bus 514 enables communications, such as data and instructions, to be exchanged between system components of the computer system 502.

The computer system 502 also includes one or more interface devices 516 such as input devices, output devices and combination input/output devices. Interface devices may receive input or provide output. More particularly, output devices may render information for external presentation. Input devices may accept information from external sources. Examples of interface devices include keyboards, mouse devices, trackballs, microphones, touch screens, printing devices, display screens, speakers, network interface cards, etc. Interface devices allow the computer system 502 to exchange information and to communicate with external entities, such as users and other systems.

The data storage 518 includes a computer readable and writeable nonvolatile, or non-transitory, data storage medium in which instructions are stored that define a program or other object that is executed by the processor 510. The data storage 518 also may include information that is recorded, on or in, the medium, and that is processed by the processor 510 during execution of the program. More specifically, the information may be stored in one or more data structures specifically configured to conserve storage space or increase data exchange performance. The data storage can include logging of counter information, tracking of compliance information, etc. Further, the data storage can include user specified time periods for first alarms, second time periods for second alarms, and total time periods for recording non-compliance.

The instructions stored in the date storage may be persistently stored as encoded signals, and the instructions may cause the processor 510 to perform any of the functions described herein. The medium may be, for example, optical disk, magnetic disk or flash memory, among other options. In operation, the processor 510 or some other controller causes data to be read from the nonvolatile recording medium into another memory, such as the memory 512, that allows for faster access to the information by the processor 510 than does the storage medium included in the data storage 518. The memory may be located in the data storage 518 or in the memory 512, however, the processor 510 manipulates the data within the memory, and then copies the data to the storage medium associated with the data storage 518 after processing is completed. A variety of components may manage data movement between the storage medium and other memory elements and examples are not limited to particular data management components. Further, examples are not limited to a particular memory system or data storage system.

Although the computer system 502 is shown by way of example as one type of computer system upon which various aspects and functions may be practiced, aspects and functions are not limited to being implemented on the computer system 502 as shown in FIG. 5. Various aspects and functions may be practiced on one or more computers having different architectures or components than that shown in FIG. 5. For instance, the computer system 502 may include specially programmed, special-purpose hardware, such as an application-specific integrated circuit (ASIC) tailored to perform a particular operation disclosed herein. While another example may perform the same function using a grid of several general-purpose computing devices running MAC OS System X with Motorola PowerPC processors and several specialized computing devices running proprietary hardware and operating systems.

The computer system 502 may be a computer system including an operating system that manages at least a portion of the hardware elements included in the computer system 502. In some examples, a processor or controller, such as the processor 510, executes an operating system. Examples of a particular operating system that may be executed include a Windows-based operating system, such as, Windows NT, 2000 (Windows ME), XP, Vista, Windows 7, 8, or RT operating systems, available from the Microsoft Corporation, a MAC OS System X operating system available from Apple Computer, one of many Linux-based operating system distributions, for example, the Enterprise Linux operating system available from Red Hat Inc., a Solaris operating system available from Sun Microsystems, or a UNIX operating systems available from various sources. Many other operating systems may be used, and examples are not limited to any particular operating system.

The processor 510 and operating system together define a computer platform for which application programs in high-level programming languages are written. These component applications may be executable, intermediate, bytecode or interpreted code which communicates over a communication network, for example, the Internet, using a communication protocol, for example, TCP/IP. Similarly, aspects may be implemented using an object-oriented programming language, such as .Net, SmallTalk, Java, C++, Ada, C# (C-Sharp), Objective C, or Javascript. Other object-oriented programming languages may also be used. Alternatively, functional, scripting, or logical programming languages may be used.

Additionally, various aspects and functions may be implemented in a non-programmed environment, for example, documents created in HTML, XML or other format that, when viewed in a window of a browser program, can render aspects of a graphical-user interface or perform other functions. For example, an administration component can render an interface in a browser to enable definition of contamination risks.

Further, various examples may be implemented as programmed or non-programmed elements, or any combination thereof. For example, a web page may be implemented using HTML while a data object called from within the web page may be written in C++. Thus, the examples are not limited to a specific programming language and any suitable programming language could be used. Accordingly, the functional components disclosed herein may include a wide variety of elements, e.g., specialized hardware, executable code, data structures or data objects, that are configured to perform the functions described herein.

In some examples, the components disclosed herein may read parameters that affect the functions performed by the components. These parameters may be physically stored in any form of suitable memory including volatile memory (such as RAM) or nonvolatile memory (such as a magnetic hard drive). In addition, the parameters may be logically stored in a propriety data structure (such as a database or file defined by a user mode application) or in a commonly shared data structure (such as an application registry that is defined by an operating system). In addition, some examples provide for both system and user interfaces that allow external entities to modify the parameters and thereby configure the behavior of the components.

According to some embodiments, hygiene protocols can be enforced as individuals enter a managed room. In other embodiments, hygiene protocols and, for example, alarms can be triggered as individuals exit a managed room. For example, hygiene stations near the exit of the room can be monitored to ensure hygiene protocols are followed.

Having thus described several aspects of at least one example, it is to be appreciated that various alterations, modifications, and improvements will readily occur to those skilled in the art. For instance, examples disclosed herein may also be used in other contexts. Such alterations, modifications, and improvements are intended to be part of this disclosure, and are intended to be within the scope of the examples discussed herein. Accordingly, the foregoing description and drawings are by way of example only.

## Claims

1. A system for managing hygiene compliance, the system comprising:
at least one processor operatively connected to a memory, the at least one processor configured to instantiate a plurality of system components;
a detection component configured to detect at least one person within a managed room;
a timer component configured to manage a threshold time period responsive to detecting the at least one person within the managed room;
a hygiene monitoring component configured to receive activation information from one or more hygiene stations; and
an alert component configured to trigger an alarm based on activation information associated with the one or more hygiene stations and the threshold time period.

2. The system according to claim 1, wherein the timer component is further configured to manage multiple time periods associated with each entry into the managed room or wherein the timer component is further configured to trigger respective timers associated with each person entering the managed room.

3. The system according to claim 2, wherein the timer component is further configured to communicate a first signal to the alert component upon expiration of a first time period.

4. The system according to claim 3, wherein the alert component is further configured to trigger a first alarm responsive to expiration of the first time period.

5. The system according to claim 2, wherein the timer component is further configured to communicate a second signal to the alert component upon expiration of a second time period.

6. The system according to claim 5, wherein the alert component is further configured to trigger a second alarm responsive to expiration of the second time period.

7. The system according to claim 1, wherein the alert component is further configured to trigger one of or both of a visual and audible alarm responsive to expiration of the threshold time period without activation of the one or more hygiene stations or where the alert component is further configured to trigger an alarm responsive to failing to detect an activation of the one or more hygiene stations within a threshold time period associated with each respective timer.

8. The system according to claim 7, wherein the audible alarm includes a reminder message announced in multiple languages.

9. A method for managing hygiene compliance, the method comprising:
detecting, by a detector, at least one person within a managed room responsive to access information;
managing, by a timer, a threshold time period responsive to detecting the at least one person within the managed room;
monitoring one or more hygiene stations for activation information; and
triggering an alarm based on the activation information associated with the one or more hygiene stations and the threshold time period.

10. The method according to claim 9, wherein managing the threshold time period includes managing multiple time periods associated with each entry into the managed room or managing respective timers associated with each person entering the managed room..

11. The method according to claim 10, further comprising generating a first signal upon expiration of a first time period.

12. The method according to claim 11, further comprising triggering a first alarm responsive to generating the first signal.

13. The method according to claim 10, further comprising generating a second signal upon expiration of a second time period.

14. The method according to claim 13, further comprising triggering a second alarm responsive to generating the second signal.

15. The method according to claim 9, further comprising triggering one of or both of a visual and audible alarm responsive to exceeding the threshold time period without activation of the one or more hygiene stations or wherein triggering the alarm includes triggering an alarm responsive to failing to detect an activation of the one or more hygiene stations within a threshold time period associated with each respective timer.
